Europäisches Patentamt

(19) European Patent Office

Office européen des brevets

(11) Publication number: **0 068 046**
**B1**

(12) **EUROPEAN PATENT SPECIFICATION**

(45) Date of publication of patent specification: **18.05.88**

(51) Int. Cl.⁴: **A 61 B 17/10**

(21) Application number: **81109835.9**

(22) Date of filing: **23.11.81**

(54) **Surgical ligating instrument.**

(30) Priority: **29.06.81 US 278258**
**29.06.81 US 278257**

(43) Date of publication of application:
**05.01.83 Bulletin 83/01**

(45) Publication of the grant of the patent:
**18.05.88 Bulletin 88/20**

(84) Designated Contracting States:
**BE CH DE FR GB IT LI NL SE**

(56) References cited:
**GB-A-2 006 021**
**GB-A-2 056 898**
**GB-A-2 074 030**
**US-A-3 837 555**

(73) Proprietor: **AMERICAN CYANAMID COMPANY**
**1937 West Main Street P.O. Box 60**
**Stamford Connecticut 06904-0060 (US)**

(72) Inventor: **Montgomery, John R.**
**715 Sasco Hill Road**
**Fairfield Connecticut 06430 (US)**
Inventor: **Brumaghim, Milton W.**
**Bear Hills Road**
**Newtown Connecticut 06470 (US)**
Inventor: **Deniega, Jose C.**
**22 Fairview Road**
**Brookfield Connecticut 06804 (US)**
Inventor: **Butter, Reinhart**
**1576 Lafayette Drive**
**Columbus Ohio 43220 (US)**

(74) Representative: **Wächtershäuser, Günter, Dr.**
**Tal 29**
**D-8000 München 2 (DE)**

Courier Press, Leamington Spa, England.

## Description

This invention relates to a surgical ligating instrument having loading, clip advancing and crimping means with two cooperating jaws. Such instrument is known from GB—20 56 898 or from US—A—3,837,555.

The improved surgical ligating instrument of the present invention is characterized in said jaws are canted and a narrow gap separates the jaws in the instrument's relaxed position so that after feeding a clip into the gap the jaws are opened by the clip and the uncrimped or partially crimped clip is held in the gap between said jaws.

A surgical ligating instrument of this invention has advantages over the known prior art. One advantage is no rectilinear motion of the jaws during crimping. The accuracy of placing the ligating clip is thus improved or may even be maximized.

The instrument can be operated "on site" with only one hand. The loading, advancing and crimping of the ligating clip(s) in the instrument is only with compression forces of the hand. The efficiency and time for ligating thus may be maximized.

Another advantage are the canted jaws. In the instrument's relaxed position, a narrow gap separates the jaws. The clip opens the jaws during loading. The jaws will thus hold the clip prior to and during a partial crimping. That is, the clip remains in the jaws if the compression force on the jaws is released before complete crimping.

The instrument may have an integral clip loading (into the jaws) and advancing means. An integral system can improve the reliability of the instrument because the number of mechanical parts is reduced. Also, an integral system can decrease or even eliminate the possibility of double loading a clip into the jaws.

In a prefered embodiment a clip injector having holding means is provided. The injector can be held adjacent to a single clip in the jaws. The injector thus prevents the clip from backing out of the jaws prior to crimping. Also, the possibility of feeding another clip (double feeding) into the jaws is eliminated. Finally, because the trigger is depressed when the injector is adjacent a single clip in the jaws, the user has visual confirmation that the injector holding means has been activated.

Preferably, a trigger lock is provided which is activated when the instrument is exhausted of clips. The user thus has manual identification that there are no clips remaining in the instrument.

Another prefered improved surgical ligating instrument of this invention has the housing in substantial parallel alignment with the probe. In this alignment, a high degree of accuracy can be obtained in the use of the instrument. For example, the orientation of the probe is in the same plane as the motion of the user's hand.

Another advantage of a specific embodiment of the invention is the compactness of the housing. A predominant portion of the housing can be easily held by the palm of a hand. This can be most important in internal surgery, for example in a body cavity, where the compactness and/or narrowness of the instrument may enable the surgeon to have a higher degree of control.

Yet another advantage of a specific embodiment of the invention is the "pointing" of the fingers as the hand grips the instrument. Specifically, a thumb and at least an index finger form a pointed profile as the hand grips the instrument. The pointing has a streamline effect which can allow the user to place the instrument as deeply as possible into a body cavity.

In a preferred embodiment of the instrument the upper portion of the housing and first activating means (for example a trigger) are in diagonal alignment with the probe. A diagonal alignment of the housing upper portion and trigger seems to counteract the optical and manual illusion that the probe is nonparallel with the housing. A diagonal alignment may also better confirm to the contour of the hand.

The crimping motion of the jaws can be substantially parallel to the compression motion of the first and the second activating means. The motion of the jaws can therefore be an extention of the squeezing motion of the user's hand. The hand/eye (more formally termed the visual/tactile) coordination of the user is therefore increased and may even be maximized. Further, another advantage is a resting area for the index finger. The resting can allow the index finger to be used as a reaction force against the first activating means being compressed by the thumb. Also, the resting area can allow the index finger to have more accuracy guiding the instrument.

Finally, the location of the second activating means can be such that a strong compressive force can be applied by the index and/or middle finger. Since either of both of these fingers tend to be the strongest, the efficiency in the use of the instrument is increased and may even be maximized.

The present invention provides a surgical ligating instrument having loading, clip advancing and crimping means with two cooperating jaws, characterized in that said jaws are canted and a narrow gap separates the jaws in the instrument's relaxed position so that after feeding a clip into the gap the jaws are opened by the clip and the uncrimped or partially crimped clip is held in the gap between said jaws.

In a preferred embodiment of the instrument said loading means load a ligating clip from the initial end of said jaws.

According to a preferred embodiment of the above described surgical ligating instrument having loading means containing a single clip; advancing means containing a plurality of clips; and crimping means said advancing means has a clip actuator, said actuator having a first and a second pawl in alternate relationship whereby said actuator can repeatedly release the frontmost clip of said plurality of clips.

A further embodiment of the instrument according to claim 1 comprises

a. a housing having a first and a second force activating and force translating means to effect a first and a second force, respectively.

b. loading means having an injector, the initial end of said injector adjacent said first translating means and the terminal end adjacent a single clip;

c. advancing means having a pair of aligned members, each member containing an external and an internal slot, the initial end of said members contained in said housing and the terminal end containing jaws; a clip actuator rotatably mounted adjacent the initial end of said jaws, said actuator having a first and a second pawl in alternate relationship; a spring, the initial end contained in said housing, a pusher at the terminal end of said spring, and a plurality of clips, the rearmost clip adjacent said pusher and the frontmost clip contained by said actuator, said spring, pusher and clips contained by the internal slots of said members, and said injector adjoining the external slots; and

d. crimping means having a crimp bar, the initial end adjacent said second translating means and the terminal end adjacent said jaws, said crimp bar holding said injector said external slots.

Other embodiments of the above described surgical ligating instrument are wherein said first activating means is a trigger; and wherein said trigger is activated by at least one thumb.

Yet other embodiment of the above described ligating instrument are wherein said first translating means is a sector gear on the terminal end of said trigger and a rack on the initial end of said injector; wherein said first translating means has at least one reduction gear such that said sector gear meshes with said reduction gear, and said reduction gear meshes with said rack; wherein said injector has holding means, said holding means comprising a pin on the initial end of said injector and a rotatable pawl contained in said housing, the distance between said pin and rotatable pawl about equal to the travel of a single clip adjacent the terminal end of said injector to said jaws whereby said injector is held adjacent to said single clip in said jaws; and wherein said second activating means on complete compression rotates said rotatable pawl such that said pin can be released.

Still other embodiments of the above described instrument are wherein said injector contains a flanged portion and said pusher contains an opening whereby said flanged portion is held by said opening after said rear most clip is crimped by said jaws; and wherein said first activating means is simultaneously locked as said flanged portion is held by said opening. The configuration of the opening is not critical provided the flanged portion can be held by the opening. A rectangular, square or circular opening can be used.

Still yet other embodiments are wherein said second activating means is a handle; wherein said handle is activated by at least one finger; and wherein said second translating means is a pin at the initial end of said crimp bar and a cam on said handle.

Still another embodiment is wherein said jaws are canted.

Another embodiment of the surgical ligating instrument has a housing containing a first and a second force activating means; and clip loading, advancing and crimping means. The improvement comprises said housing in substantial parallel alignment with said loading, advancing and crimping means.

One embodiment of this improved instrument is wherein a predominant portion of said housing can be essentially held by the palm of a hand; and wherein said first and second force activating means can be operated respectively by a thumb, and by at least one figner in a nonextended position.

Another embodiment of the improved instrument is wherein said loading, advancing and crimping means are contained in a probe, the terminal end of said probe containing jaws; wherein said first and second activating means are in opposing relationship and adjacent said probe; and wherein a thumb is placed on said first activating means and at least an index finger is placed on said second activating means such that said thumb, and at least said index finger form a pointed profile.

Still another embodiment of the improved instrument is wherein said housing has a curved portion between said second activating means and said probe; wherein an index finger is placed on said curved portion; and wherein a thumb is placed on said first activating means and at least a middle finger is placed on said second activating means such that said thumb, said index finger and at least said middle finger form a pointed profile.

A further embodiment of the improved instrument is wherein the crimping motion of said jaws is substantially parallel to the compression motion of said first and second activating means.

Description of the drawings

Figure 1 is a broken cut-away side view of the instrument housing showing the activating and translating means in a rest position;

Figures 2 and 3 are cut-away bottom and partially broken perspective views of Figure 1 showing the loading, clip advancing and crimping means;

Figure 4 is a broken cut-away side view of the instrument housing showing the first activating and translating means in the compressed position;

Figures 5 and 6 are cut-away bottom and partially broken perspective views of Figure 4 showing the position of the injector, jaws and clip actuator;

Figure 7 is a broken cut-away side view of the instrument housing showing the second activating and translating means in the compressed position; and

Figures 8 and 9 are cut-away bottom and partially broken perspective views of Figure 7 showing the loading, clip advancing and crimping means.

Figure 10 is a partially broken top plan view of a surgical ligating instrument of this invention;

Figure 11 is a partially broken side elevation of Figure 10;

Figure 12 is a bottom plan view of Figure 11;

Figure 13 is a back plan view of Figure 11;

Figures 14 to 16 are alternative side elevation views of a surgical ligating instrument of Figure 11; and

Figure 17 is a broken and exploded top plan view showing the probe of Figure 10.

Detailed description

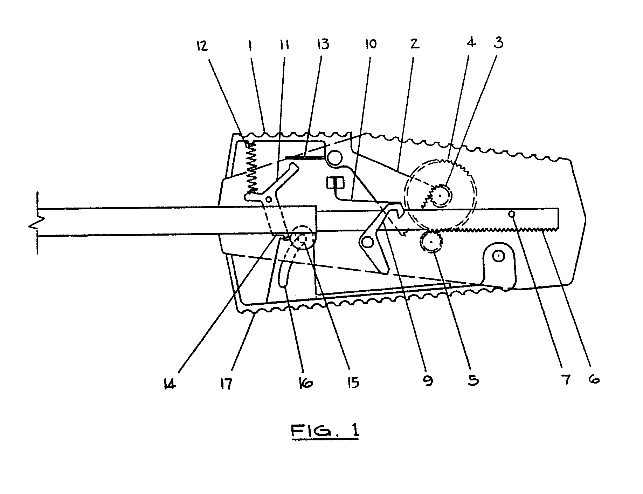

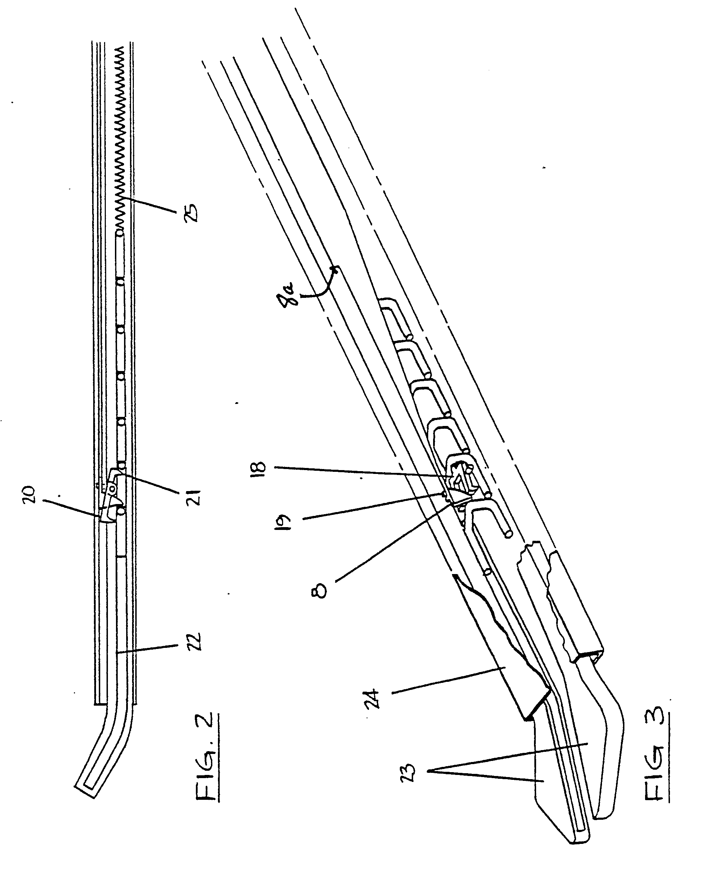

Figure 1 shows the parts in the housing of the device. Figure 2 is a cross-section and Figure 3 is a schematic showing the parts in the probe. Referring to Figures 1 to 3, clip injector 8 is rigidly attached to rack 6. Alternatively, injector 8 can be manufactured as one part with the rack 6 contained on the initial end. Rack 6 carries pin 7.

Reduction gears 3 and 4 are keyed together to form a cluster. Alternatively, gears 3 and 4 can be manufactured as one part. Sector gear 2 meshes with gear 3, and gear 4 meshes with pinion gear 5. Rack 6 meshes with gear 5.

Spring 25 is compressed between a pusher and rack 6. The rearmost clip of a column of clips is adjacent the pusher. The spring is thus applying a constant force to urge a plurality of clips forward and the rack 6 backward.

A plurality of clips are contained in an internal slot that runs throughout the length of a pair of aligned members 23. The terminal end of the members 23 contain jaws.

Pin 15 is contained on and therefore is carried by the motion of crimp bar 24. The pin 15 rides on cam surface 16.

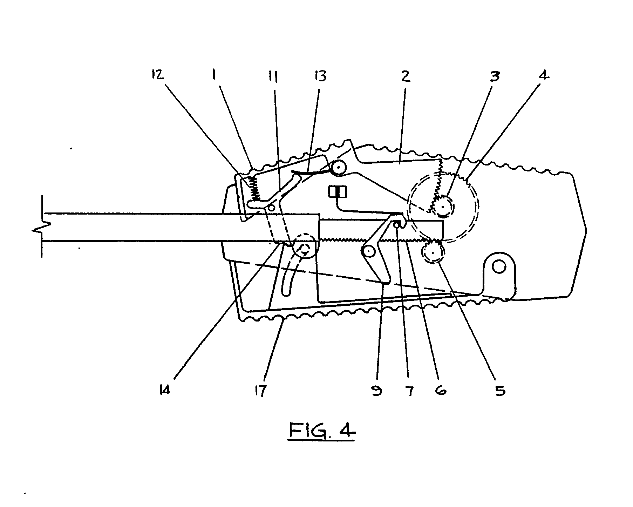

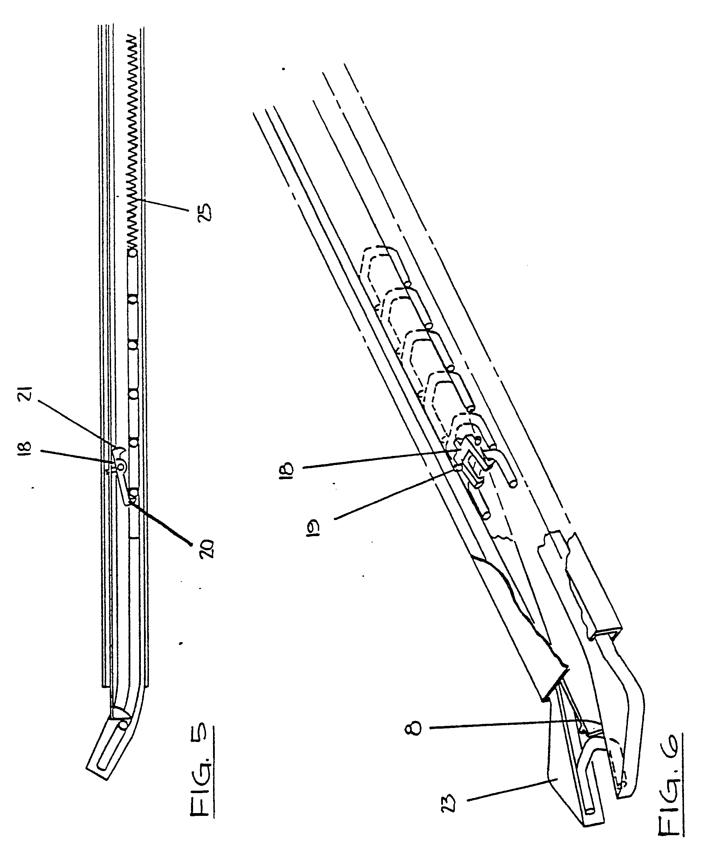

Referring to Figures 4 to 6, a single clip is pushed to the jaws by the clip injector 8. This is accomplished by compressing feed trigger 1 which turns sector gear 2 counter-clockwise and cluster gears 3 and 4 clockwise. Pinion gear 5 turns counter-clockwise driving rack 6 and clip injector 8 forward against spring 25, pushing the lead clip to the tip of the jaws (as shown in Figures 5 and 6). The clip injector 8 is held in the forward position by a rotatable pawl 9 which is contained in the housing and which catches pin 7.

Near the end of the forward stroke of the clip injector 8, a detail 8a (shown in Fig. 3) on the clip injector pushes the actuator stem 19 causing the actuator 18 to rotate forward dropping pawl 20 and lifting pawl 21. Since the column of clips is constantly being urged forward by spring 25, the clips which were being held back by pawl 21 (as shown e.g. in Figure 2) are now free to slide forward until the lead clip is stopped by pawl 20.

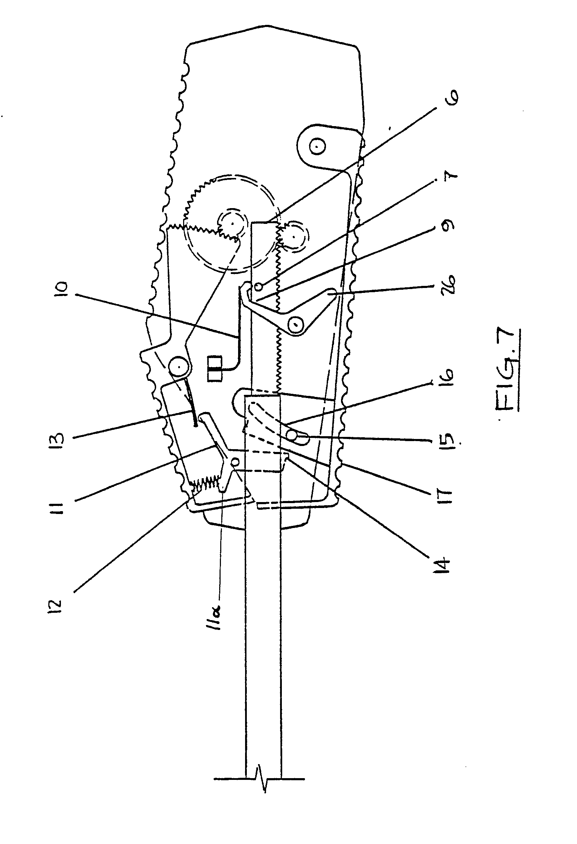

Referring to Figures 4 and 7 crimp bar interlock 11 prevents the handle 17 from being depressed until trigger 1 is completely compressed. Compression spring 12 applies a constant force to urge a first lever arm 11a to turn counter-clockwise. When the trigger 1 is approximately one-half compressed leaf spring 13 will hit a second lever arm 11b. Because the force of leaf spring 13 is greater than spring 12 and because the effective length of the second lever arm is longer than the first lever arm, leaf spring 13 overrides compression spring 12. Therefore, crimp bar interlock 11 tends to turn clockwise.

If there is pressure on the crimp bar handle 17 when trigger 1 is depressed, dog 14 does not disengage. Crimp bar handle 17 is thus presented from being compressed. When the pressure on the crimp bar handle 17 is released, dog 14 disengages. This action causes crimp bar interlock 11 to turn clockwise. The cam plate 16 carried by the crimp bar handle 17 is thus activated.

The clip which has been in the jaws is now ready to be crimped. This is accomplished by completely compressing the crimp bar handle 17.

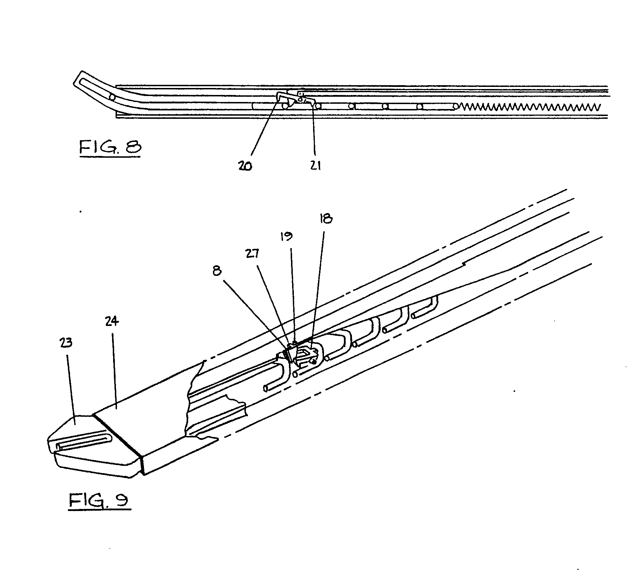

Referring specifically to Figures 7 to 9, as the crimp bar handle 17 is being compressed, crimp bar 24 is pushed forward by the pin 15 acting on cam 16. The front end of the crimp bar 24 cams on the incline planes at the jaws 23. The camming causes the two jaws to come together crimping the clip.

As the crimp bar handle 17 approaches a complete compression, it contacts lever 26 pushing pawl 9 up against leaf spring 10 and releasing pin 7. The rack 6 is now free to slide back pulling with it the injector 8. As shown in Figure 9, tab 27 hits stem 19, rotating clip actuator 18. This drops pawl 21 to hold back the column of clips that is being urged forward by spring 25, and lifts pawl 20 to clear the path for the lead clip. The front end of the clip injector 8 is flexible such that it cams over and gets behind the lead clip. The injector 8 can contain a flanged portion at the terminal end and the pusher can contain an opening such that the flanged portion is held by the opening after the rearmost clip is crimped by the jaws. The trigger 1 is thus locked when the clips are exhausted.

In the relaxed position, a narrow gap separates the jaws 23. The clip opens the jaws during loading. The jaws can thus hold the clip before crimping, and also during partial crimping, that is, if the crimp bar handle 17 is released before complete compression.

Referring to Figures 11 and 14 to 16, the surgical ligating instrument has a housing 30. The housing contains a first activating means, for example a trigger 1, and a second activating means, for example a handle 17. The first and the second force translating means contained in the housing 30 is activated by the trigger 1 and the handle 17, respectively. Either one or both of the force translating means can be the mechanisms shown in Figures 1, 4 and 7.

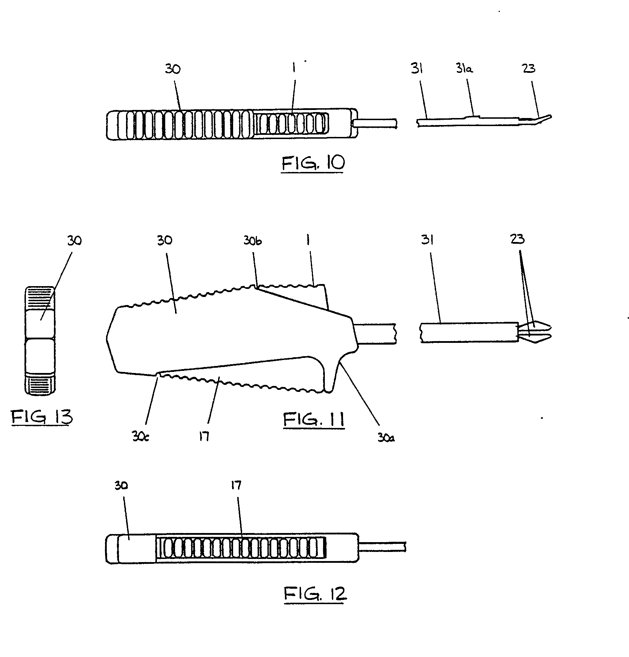

Referring to Figures 10, 11 and 17, the instrument has a probe 31. The initial end of the loading, advancing and crimping means is contained in the housing 30.

The housing 30 is in substantial parallel alignment with the probe 31. Preferably, the first activating means 1 and the upper portion of the housing are in a diagonal alignment with the probe 31. A diagonal alignment seems to counteract the optical and manual illusion that the probe is nonparallel with the housing and may better conform to the contour of the hand.

Preferably, the size of the housing is such that a predominant portion of the housing can be essentially held by the palm of a hand.

The terminal end of the probe 31 contains jaws 23.

Referring to Figure 11, in one embodiment the housing has a curved portion 30a between the second activating means 17 and the probe 31. An index finger can be placed on the curved portion.

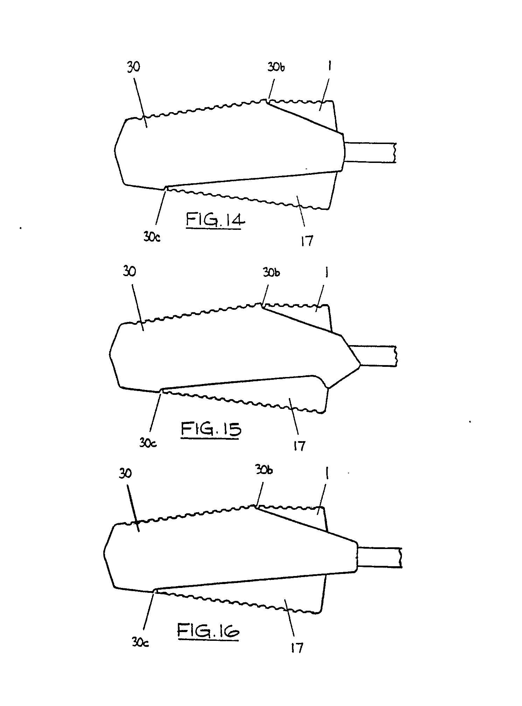

Figures 14 to 16 are alternative embodiments of the surgical instruments housing shown in Figures 10 to 13. The description of the housing of Figures 10 to 13 essentially corresponds to Figures 14 to 16.

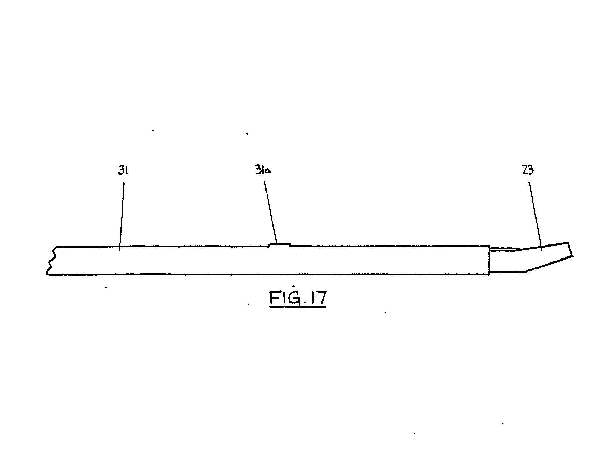

Figure 17 describes in more detail the probe 31. The optional raised portion 31a can be a transparent material and is adjacent to the initial end (not shown) of the jaws 23 in the probe. The raised portion gives a visual identification to the user that clips are/are not remaining in the instrument.

Referring to Figure 10 to 15 a scalloped configuration in the upper portion of the housing and the first activating means and in the lower portion of the second activating means is preferred. The scalloping assists the user to grip the housing and to compress the first and the second activating means. The partial and broken scalloping shown in Figures 10 to 12 are for clarity of illustration. It is to be understood that the scalloping in Figures 10 and 12 is consistent with Figure 11. It is also to be understood that the probe shown in Figure 11 is identical in Figures 14 to 16.

A notch 30b between the first activating means and the upper portion of the housing, and a notch 30c between the second activating means and the lower portion of the housing reduces or may even eliminate the possibility that the hand and/or fingers of the user are punched when the first and the second activating means are returning to the relaxed position.

## Claims

1. A surgical ligating instrument having loading, clip advancing and crimping means with two cooperating jaws, characterized in that said jaws (23) are canted and a narrow gap separates the jaws (23) in the instrument's relaxed position so that after feeding a clip into the gap the jaws (23) are opened by the clip and the uncrimped or partially crimped clip is held in the gap between said jaws (23).

2. An instrument according to Claim 1 wherein said loading means (8) load a ligating clip from the initial end of said jaws (23).

3. An instrument according to claim 1, characterized in that said advancing means has a clip actuator (18), said actuator having a first and a second pawl (20;21) in alternate relationship whereby said actuator can repeatedly release the frontmost clip of said plurality of clips.

4. An instrument according to claim 1 comprising
   a. a housing having a first and a second force activating and force translating means to effect a first and a second force, respectively;
   b. loading means having an injector, the initial end of said injector adjacent said first translating means and the terminal end adjacent a single clip;
   c. advancing means having a pair of aligned members, each member containing an external and an internal slot, the initial end of said members contained in said housing and the terminal end containing jaws (23), a clip actuator (18) rotatably mounted adjacent the initial end of said jaws, said actuator having a first and a second paw (20; 21) in alternate relationship; a spring, the initial end contained in said housing, a pusher at the terminal end of said spring, and a plurality of clips, the rearmost clip adjacent said pusher and the frontmost clip contained by said actuator, said spring, pusher and clips contained by the internal slots of said members, and said injector adjoining the external slots; and
   d. crimping means having a crimp bar, the initial end adjacent said second translating means and the terminal end adjacent said jaws, said crip bar holding said injector on said external slots.

5. An instrument according to Claim 4 wherein said first activating means is a trigger (1).

6. An instrument according to Claim 5 wherein said first translating means is a sector gear (2) on the terminal end of said trigger and a rack (6) on the initial end of said injector (8).

7. An instrument according to Claim 6 wherein said first translating means has at least one reduction gear (3,4) such that said sector gear (2) meshes with said reduction gear (3,4) and said reduction gear meshes with said rack (6).

8. An instrument according to Claim 6 wherein said injector (8) has holding means, said holding means comprising a pin (7) on the initial end of said injector and a rotatable pawl (9) contained in said housing, the distance between said pin and rotatable pawl about equal to the travel of a single clip adjacent the terminal end of said injector to said jaws (23) whereby said injector is held adjacent to said single clip in said jaws.

9. An instrument according to Claim 8 wherein said second activating means on complete compression rotates said rotatable pawl (9) such that said pin (7) can be released.

10. An instrument according to Claim 4 or 5 or 9 wherein said injector (8) contains a flanged portion and said pusher contains an opening whereby said flanged portion is held by said opening after said rearmost clip is crimped by said jaws.

11. An instrument according to Claim 10 wherein said first activating means is sumultaneously locked as said flanged portion is held by said opening.

12. An instrument according to Claim 4 or 6 or 9 wherein said second activating means is a handle (17).

13. An instrument according to Claim 12 wherein said second translating means is a pin at the initial end of said crimp bar and a cam on said handle.

14. An instrument according to Claim 4 or 6 or 13 wherein said jaws are canted.

15. An instrument according to claim 1 having a housing containing a first and a second force activating means (1,17); and clip loading, advancing and crimping means, the improvement comprising said housing in substantial parallel alignment with said loading, advancing and crimping means.

16. An instrument according to Claim 15 wherein said loading, advancing and crimping means are contained in a probe (31) the terminal end of said probe containing jaws (23).

17. An instrument according to Claim 16 wherein said first and second activating means are in opposing relationship and adjacent said probe (31).

18. An instrument according to Claim 17 wherein a thumb is placed on said first activating means (1) and at least an index finger is placed on said second activating means (17) such that said thumb, and at least said index finger form a pointed profile.

19. An instrument according to Claim 16 or 17 wherein said housing has a curved portion between said second activating means (17) and said probe (31).

20. An instrument according to Claim 16 or 17 or 19 wherein the crimping motion of said jaws (23) is substantially parallel to the compression motion of said first and second activating means (1,17).

**Patentansprüche**

1. Chirurgisches Ligaturinstrument mit Einrichtungen zum Klammerladen, Klammervorschub und Klammerbiegen mit zwei zusammenwirkenden Backen, dadurch gekennzeichnet, daß die Bakken (23) abgeschrägt sind und in der relaxierten Position des Instruments ein enger Spalt die Backen (23) trennt, so daß nach dem Einführen einer Klammer in den Spalt die Bakken (23) durch die Klammer geöffnet werden und die ungekrümmte oder teilweise gekrümmte Klammer in dem Spalt zwischen den Backen (23) gehalten wird.

2. Instrument gemäß Anspruch 1, wobei die Ladeeinrichtung (8) eine Ligaturklammer vom Anfangsende der Backen (23) lädt.

3. Instrument gemäß Anspruch 1, dadurch gekennzeichnet, daß die Vorschubeinrichtung einen Klammerbetätiger (18) umfaßt, wobei der Betätiger eine erste und eine zweite Klaue (20;21) in alternierer Beziehung aufweist, wobei der Betätiger die vorderste Klammer von einer Vielzahl von Klammer wiederholt freigeben kann.

4. Instrument gemäß Anspruch 1, umfassend

(a) ein Gehäuse mit einer ersten und einer zweiten Kraftaktivierungs- und Kraftübertragungseinrichtung zur Bewirkung einer ersten bzw. einer zweiten Kraft;

(b) Ladeeinrichtungen mit einem Injektor, wobei das Anfangsende des Injektors der ersten Kraftübertragungseinrichtung benachbart ist und das terminale Ende einer einzelnen Klammer benachbart ist;

(c) Vorschubeinrichtungen mit einem Paar ausgerichteter Organe, wobei jedes Organ einen externene und einen internen Schlitz enthält, das Anfangsende jedes Organs in dem Gehäuse enthalten ist und das terminal Ende Backen (23) enthält; einen Klammerbetätiger (18), der drehbar benachbart dem Anfangsende der Backen befestigt ist, wobei der Betätiger eine erste und eine zweite Klaue (20;21) in alternierender Beziehung aufweist; eine Feder, deren Anfangsende in dem Gehäuse enthalten ist, einen Drücker am terminalen Ende der Feder und eine Vielzahl von Klammern, wobie die hinterste Klammer dem Drücker benachbart ist und die vorderste Klammer mit dem Betätiger zusammenwirkt, wobei bei die Feder, der Drücker, und die Klammern in den internen Schlitzen der Organe enthalten sind und der Injektor an die externen Schlitze angrenzt; und

(d) Biegeeinrichtungen mit einer Biegestange, deren Anfangsende der zweiten Kraftübertragungseinrichtung benachbart ist und deren terminales Ende den Backen benachbart ist, wobei die Biegestange den Injektor auf den externen Schlitzen hält.

5. Instrument gemäß Anspruch 4, wobei die erste Kraftübertragungseinrichtung ein Auslösehebel (1) ist.

6. Instrument gemäß Anspruch 5, wobei die erste Kraftübertragungseinrichtung ein Zahnradsegment (2) am terminalen Ende des Auslösehebels und eine Zahnstange (6) am Anfangsende des Injektors (8) umfaßt.

7. Instrument gemäß Anspruch 6, wobei die erste Kraftübertragungseinrichtung mindestens ein Untersetzungsgetriebe (3,4) aufweist, und zwar derart, daß das Zahnradsegment (2) mit dem Untersetzungsgetriebe (3,4) kämmt und das Untersetzungsgetriebe mit der Zahnstange (6) kämmt.

8. Instrument gemäß Anspruch 6, wobei der Injektor (8) Halteeinrichtungen aufweist, wobei die Halteeinrichtungen einen Stift (7) am Anfangsende des Injektors umfassen sowie eine drehbare Klaue (9), die in dem Gehäuse enthalten ist, wobei der Abstand zwischen dem Stift und der drehbaren Klaue etwa ebenso groß ist wie der Weg einer einzelnen Klammer, die dem terminalen Ende des Injektors benachbart ist, bis zu den Backen (23) wobei der Injektor benachbart der einzelnen Klammer in den Backen gehalten wird.

9. Instrument gemäß Anspruch 8, wobei die zweite Kraftübertragungseinrichtung bei vollständigem Zusammendrücken die drehbare Klaue (9) dreht, und zwar derart, daß der Stift (7) freigegeben werden kann.

10. Instrument gemäß Anspruch 4 oder 5 oder 9, wobei der Injektor (8) einen Flanschbereich aufweist und der Drücker eine Öffnung enthält, wobei der Flanschbereich durch die Öffnung gehalten wird, nachdem die hinterste Klammer durch die Backen gebogen worden ist.

11. Instrument gemäß Anspruch 10, wobei die erste Aktivierungseinrichtung gleichzeitig verriegelt wird, sobald der Flanschbereich durch die Öffnung gehalten wird.

12. Instrument gemäß Anspruch 4 oder 6 oder 9, wobei die zweite Aktivierungseinrichtung ein Handgriff (17) ist.

13. Instrument gemäß Anspruch 12, wobei die zweite Kraftübertragungseinrichtung ein Stift am Anfangsende der Biegestange und eine Nocke an dem Handgriff umfaßt.

14. Instrument gemäß Anspruch 4 oder 6 oder 13, wobei die Backen abgeschrägt sind.

15. Instrument gemäß Anspruch 1 mit einem Gehäuse, enthaltend eine erste und eine zweite Kraftaktivierungseinrichtung (1,17); und Klammerlade-, Vorschub- und Biegeeinrichtungen, wobei die Verbesserung darin besteht, daß das Gehäuse und die Lade-, Vorschub- und Biegeeinrcihtungen im wesentlichen parallel ausgerichtet sind.

16. Instrument gemäß Anspruch 15, wobei die Lade-, Vorschub- und Biegeeinrichtungen in einer Sonde (31) enthalten sind, wobei das terminale Ende der Sonde Backen (23) enthält.

17. Instrument gemäß Anspruch 16, wobei die ersten und zweiten Aktivierungseinrichtungen in gegenüberliegender Beziehung und benachbart der Sonde (31) angeordnet sind.

18. Instrument gemäß Anspruch 17, wobei ein Daumen auf die erste Aktivierungseinrichtung (1) plaziert wird und mindestens ein Zeigefinger auf die zweite Aktivierungseinrichtung (17) plaziert wird derart, daß der Daumen und mindestens de Zeigefinger ein zugespitztes Profil bilden.

19. Instrument gemäß Anspruch 16 oder 17, wobei das Gehäuse einen gekrümmten Abschnitt zwischen der zweiten Aktivierungseinrichtung (17) und der Sonde (31) aufweist.

20. Instrument gemäß Anspruch 16 oder 17 oder 19, wobei die Biegebewegung der Backen (23) im wesentlichen parallel zur Drückbewegung von ersten und zweiter Aktivierungseinrichtung (1, 17) verläuft.

**Revendications**

1. Instrument pour ligatures chirurgicales possédant des moyens de chargement, d'avance et de sertissage de pinces, comportant deux mâchoires coopérant entre elles, caractérisé en ce que les mâchoires (23) sont obliques et qu'une fente étroite sépare les mâchoires (23) dans la position relâchée de l'instrument, de sorte que lorsqu'on a introduit une pince dans la fente, les mâchoires (23) s'ouvrent sous l'action de la pince et que la pince non encore sertie ou partiellement sertie est tenue dans la fente formée entre les mâchoires (23).

2. Instrument selon la revendication 1, dans lequel les moyens de chargement (8) chargent une pince de ligature en partant de l'extrémité initiale des mâchoires (23).

3. Instrument selon la revendication 1, caractérisé en ce que les moyens d'avancement comprennent un actionneur de pinces (18), cet actionneur possédant un premier cliquet et un deuxième cliquet (20;21) qui sont dans des positions alternées, de sorte que l'actionneur peut répétitivement relâcher la pince extrême avant de la série de pinces.

4. Instrument selon la revendication 1 comprenant:

a) un boîtier possédant des premiers et des deuxièmes moyens d'actionnement à force et de transmission de force, qui sont respectivement destinés à développer une première force et une deuxième force;

b) des moyens de chargement qui possèdent un injecteur, l'extrémité initiale de l'injecteur étant adjacente aux premiers moyens de transmission et l'extrémité terminale étant adjacente à une seule pince;

c) des moyens d'avancement possédant une paire d'éléments alignés, chaque élément présentant une rainure extérieure et une rainure intérieure, l'extrémité initial de ces éléments étant contenue dans le boîtier et l'extrémité terminale contenant les mâchoires (23); un actionneur de pinces (18) qui est monté rotatif à proximité de l'extrémité initiale des mâchoires, l'actionneur possédant un premier cliquet et un deuxième cliquet (20;21) qui sont dans des positions alternées; un ressort dont l'extrémité initiale est contenue dans le boîtier, un poussoir situé à l'extrémité terminale du ressort, et une série de pinces, la pince extrême arrière étant adjacente au poussoir et la pince extrême avant étant contenue dans l'actionneur, le ressort, le poussoir et lesdites pinces, et l'injecteur étant adjacent aux rainures extérieures; et

d) des moyens de sertissage possédant une barre de sertissage dont l'extrémité initiale est adjacente aux deuxièmes moyens de transmission et l'extrémité terminale adjacente aux mâchoires, la pince de sertissage maintenant l'injecteur sur les rainures extérieures.

5. Instrument selon la revendication 4, dans lequel les premiers moyens d'actionnement sont constitués par une gâchette (1).

6. Instrument selon la revendication 5, dans lequel lesdits premiers moyens de transmission sont constitués par un secteur denté (2) prévu sur l'extrémité terminale de la gâchette et une crémaillère (6) prévue sur l'extrémité initiale de l'injecteur (8).

7. Instrument selon la revendication 6, dans lequel les premiers moyens de transmission comprennent au moins une roue dentée de réduction (3,4), de sorte que le secteur denté (2) engrène avec la roue dentée de réduction (3,4) et que la roue dentée de réduction engrène avec la crémaillère (6).

8. Instrument selon la revendication 6, dans lequel l'injecteur possède des moyens de retenue comprenant un ergot (7) situé sur l'extrémité initiale de l'injecteur et un cliquet pivotant (9) contenu dans le boîtier, la distance entre l'ergot et le cliquet pivotant étant à peu près égale à la course qu'une pince seule adjacente à l'extrémité terminale de l'injecteur parcourt pour atteindre lesdites mâchoires (23), de sorte que l'injecteur est maintenu adjacent à la pince seule dans les mâchoires.

9. Instrument selon la revendication 8, dans lequel les moyens d'actionnement font tourner le cliquet pivotant (9), lorsqu'ils sont entièrement comprimés, de sorte que l'ergot (7) peut être relâché.

10. Instrument selon la revendication 4, 5 ou 9, dans lequel l'injecteur (8) comprend une ailette et le poussoir présente une ouverture, de sorte que l'ailette est retenue par l'ouverture, après que la pince extrême arrière a été sertie par les mâchoires.

11. Instrument selon la revendication 10, dans lequel les premiers moyens d'actionnement sont simultanément verrouillés lorsque l'ailette est maintenue par l'ouverture.

12. Instrument selon la revendication 4, 6 ou 9, dans lequel les deuxièmes moyens d'actionnement sont constitués par une poignée (17).

13. Instrument selon la revendication 12, dans lequel les deuxièmes moyens de transmission sont constitués par un ergot prévu sur l'extrémité initiale de la barre de sertissage et une came portée par la poignée.

14. Instrument selon la revendication 4, 6 ou 13, dans lequel les mâchoires sont obliques.

15. Instrument selon la revendication 1, possédant un boîtier qui contient des premiers et des deuxièmes moyens d'actionnement à force (1,17); et des moyens de chargement, d'avancement et de sertissage des pinces, dans lequel le boîtier est aligné sensiblement parallèlement aux moyens de chargement, d'avancement et de sertissage.

16. Instrument selon la revendication 15, dans lequel les moyens de chargement, d'avancement et de sertissage sont contenus dans une sonde (31), l'extrémité terminale de la sonde renfermant les mâchoires (23),

17. Instrument selon la revendication 16, dans lequel les premiers et deuxièmes moyens d'actionnement sont dans des positions opposées et adjacentes à la sonda (31).

18. Instrument selon la revendication 17, dans lequel on place le pouce sur les premiers moyens d'actionnement (1) et au moins l'index sur les deuxièmes moyens d'actionnement (17) de telle manière que le pouce et au moins l'index forment un profil en pointe.

19. Instrument selon la revendication 16 ou la revendication 17, dans lequel le boîtier possède une partie courbe comprise entre les deuxièmes moyens d'actionnement (17) et la sonde (31).

20. Instrument selon la revendication 16, 17 ou 19, dans lequel le mouvement de sertissage des mâchoires (23) est à peu près parallèle au mouvement de compression des premiers et deuxièmes moyens d'actionnement (1,17).

12  1  11  13  10  2  4  3

14  17  16  15  9  5  7  6

FIG. 1

1

FIG. 2

FIG 3

FIG. 4

0 068 046

FIG. 5

FIG. 6

4

FIG.7

FIG. 8

20    21

FIG. 9

23    24    8    27    19    18

30    1          31   31a    23

FIG. 10

30        30    30b    1         31        23

FIG 13          FIG. 11

30c   17        FIG. 12

30a

30    17

FIG. 12

FIG.14

FIG.15

FIG.16

31          31a                    23

FIG.17